# FASCICULE DE BREVET EUROPEEN

(11) **EP 0 643 056 B1**
(45) Date de publication et mention de la délivrance du brevet: **16.02.2000**
(21) Numéro de dépôt: 94402021.3
(22) Date de dépôt: 12.09.1994
(51) Int. Cl.: C07D 319/06, C07D 207/40

(54) **Procédé de préparation stéréospécifique de dérivés de 1,3-dioxane-4,6-diones précurseurs de dérivés d'acides tétramiques chiraux**
Verfahren zu stereospezifistischer Herstellung von 1,3-Dioxan-4,6-dion Derivaten, Vorläufer von tetramischen Säuren Derivaten mit Chiralstelle
Process for the stereospecific preparation of 1,3-dioxan-4,6-dion derivatives, precursors of chiral tetramic acids derivatives

(30) Priorité: 15.09.1993 FR 9310965
(43) Date de publication de la demande: 15.03.1995
(73) Titulaire: ISOCHEM, 92230 Gennevilliers (FR)
(72) Inventeur: Loffet, Albert, F-75015 Paris (FR); Martinez, Jean, F-34570 Saussan (FR); Fehrentz, Jean-Alain, F-34400 Saint Nazaire de Pezan (FR); Winternitz, Philippe-Francois, F-34000 Montpellier (FR)
(74) Mandataire: Pech, Bernard

(56) Documents cités:
- JOURNAL OF MEDICINAL CHEMISTRY, vol.32, no.7, 1989, WASHINGTON US pages 1571 - 1576 J. MAIBAUM ET AL. 'SYNTHESIS OF THE NOVEL PY-(BEZYLOXYMETHYL)-PROTECTED HISTIDINE ANALOGUE OF STATINE.'
- JOURNAL OF THE CHEMICAL SOCIETY, PERKIN TRANSACTIONS 1, no.1, 1987, LETCHWORTH GB pages 1177 - 1182 P. JOUIN, B. CASTRO 'STEREOSPECIFIC SYNTHESIS OF N-PROTECTED STATINE AND ITS ANALOGUES VIA CHIRAL TETRAMIC ACID.'
- TETRAHEDRON LETTERS, vol.34, no.42, Août 1993, OXFORD GB pages 6705 - 6708 JIANJUN JIANG ET AL. 'COMPARATIVE STUDY OF SELECTED REAGENTS FOR CARBOXYL ACTIVATION.'
- J.A.C.S. vol. 112, no. 20, pages 7414 - 7416

## Description

L'invention concerne un procédé de préparation stéréospécifique de dérivés de 5-(1-hydroxy-2-uréthane-éthylidène)-2,2-diméthyl-1,3-dioxane-4,6-diones permettant d'obtenir des dérivés d'acides tétramiques chiraux. Ces dérivés d'acides tétramiques sont très utiles pour synthétiser des 3-hydroxy-4-amino-acides tels que la statine et ses analogues qui sont des amino-acides très importants en chimie thérapeutique. Les composés de cette famille sont notamment des constituants caractéristiques des inhibiteurs de protéases acides et également des depsipeptides.

Plusieurs synthèses de statine et de ses analogues ont été décrites. Les plus communément utilisées sont basées sur la réaction de condensation aldolique d'énolates d'esters et d'α-acylamino-aldéhydes (P.G.M. Wuts, Synthesis, 1989, 951).

Ces méthodes présentent un certain nombre d'inconvénients tels que la racémisation ou l'obtention de diastéréoisomères qu'il faut ensuite séparer ou bien des réactifs peu courants sont utilisés.

Une autre voie a été proposée qui permet de préparer la statine et ses analogues au moyen d'intermédiaires qui sont des acides tétramiques chiraux (notamment P. Jouin et al, J. Chem. Soc. Perkin Trans, 1, 1987, 1177-1182). Ceux-ci sont obtenus par condensation d'un acide aminé N-protégé chiral avec l'acide de Meldrum en présence de chloroformiate d'isopropényle et de 4-N,N-diméthylaminopyridine suivie d'une cyclisation du dérivé dioxane obtenu. Si cette solution permet de conserver la stéréospécificité, elle n'est cependant pas très économique ni très facile à mettre en oeuvre. Le chloroformiate d'isopropényle est un réactif cher et instable. Les conditions réactionnelles sont très rigoureuses. Le chloroformiate d'isopropényle doit être additionné lentement dans une solution des autres réactifs maintenue à une température très basse voisine de - 5° C. Tout changement dans le mode opératoire, par exemple de l'ordre d'introduction des réactifs, de la température, des quantités respectives des réactifs, conduit à une baisse des rendements.

Selon la publication du J. Med. Chem., 1989, 32, 1571-1576 par J. Maibaum et al, le dérivé dioxane-dione de l'histidine dont un atome d'azote du cycle est protégé par un groupe benzyloxyméthyle a été préparé en utilisant le dicyclohexylcarbodiimide (DCC) à la place du chloroformiate d'isopropényle. La réaction a une durée très longue, de 24 heures. Seul ce dérivé d'histidine a été préparé. Pour les auteurs précédemment cités, P. Jouin et al, le DCC ne permet pas d'obtenir, avec un rendement satisfaisant, les dioxane-diones.

La présente invention a pour but de remédier à ces inconvénients et a pour objet un procédé de préparation des intermédiaires dioxanes qui est économique, simple, rapide et qui permet de conserver la chiralité du carbone asymétrique du dérivé d'amino-acide de départ.

Selon l'invention, on prépare des dérivés de 5-(1-hydroxy-2-uréthane-éthylidène)-2,2-diméthyl-1,3-dioxane-4,6-diones précurseurs d'acides tétramiques chiraux N-protégés au moyen de l'acide de Meldrum, par réaction des N-carboxy-anhydrides d'α-amino-acides N-uréthane-protégés (UNCA) avec l'acide de Meldrum, dans un milieu solvant organique inerte, en présence d'une quantité supérieure à 2 équivalents par rapport au N-carboxy-anhydride d'au moins une amine tertiaire choisie parmi les amines tertiaires dont un radical au moins est aliphatique ou cycloaliphatique. Les autres radicaux des amines sont des radicaux très variés, identiques ou différents, tels que par exemple des radicaux aliphatiques, cycloaliphatiques, araliphatiques, hydrocarbonés aromatiques ou hétérocycliques aromatiques ou non ou des radicaux qui réunis forment avec l'atome d'azote un hétérocycle pouvant comprendre d'autres hétéroatomes.

Le schéma réactionnel est le suivant : 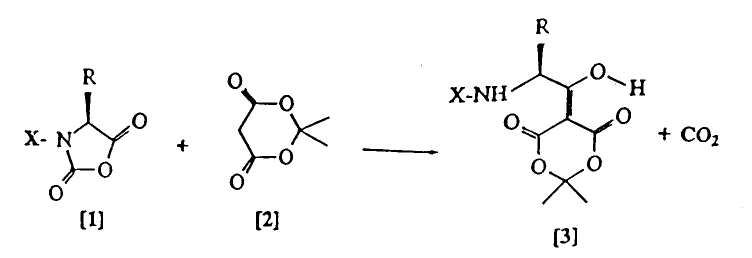 R représentant le reste d'un α-amino-acide et X représentant le groupe protecteur formant la fonction uréthane.

Ces dérivés dioxane-diones (3) peuvent ensuite être transformés de façon connue par exemple comme décrit dans les articles de P. Jouin et al précédemment cité ou de J. Poncet, J. Chem. Soc. Perkin Trans, 1, 1990, 611, tout d'abord en dérivés d'acides tétramiques N-protégés (4) qui sont réduits pour donner des dérivés de 4-hydroxy-pyrrolidine-2-ones (5). Par hydrolyse régiosélective de ces derniers composés, on obtient des diastéréoisomères purs de 3-hydroxy-4-amino-acides (6), tels que la statine ou un analogue, N-protégés.

Le schéma réactionnel de cette transformation est le suivant :

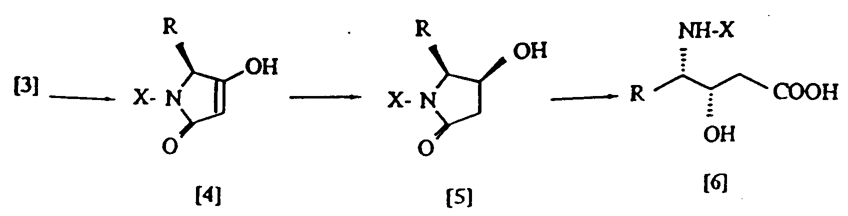

Les N-carboxy-anhydrides d'α-amino-acides N-uréthane-protégés (1) (UNCA) utilisés comme matières de départ sont des composés connus stables qui se trouvent dans le commerce ou qui peuvent se préparer comme décrit dans l'article du J. Am. chem. Soc., vol 112, No 20, 1990, 7414-7416.

Lorsque les chaînes latérales des α-aminoacides qui forment les anhydrides portent des groupes fonctionnels, ceux-ci sont généralement protégés par les groupes protecteurs habituels en synthèse peptidique.

La fonction amine de l'anhydride est protégée par un des groupes protecteurs de cette fonction habituellement utilisés en synthèse peptidique et formant une fonction uréthane. Comme groupes préférés, on peut citer les groupes (9-fluorénylméthyl)oxycarbonyle (Fmoc), tert-butyloxy-carbonyle (Boc), benzyloxycarbonyle (Z) et allyloxycarbonyle (Alloc).

L'acide de Meldrum (malonate d'isopropylidène) est un composé connu qui se trouve dans le commerce.

On a trouvé que l'on pouvait faire réagir très facilement les N-carboxy-anhydrides d'α-amino-acides N-uréthane protégés avec l'acide de Meldrum, pour obtenir les dérivés dioxane-diones précurseurs des dérivés d'acides tétramiques, tout en conservant la chiralité du composé de départ.

La présence d'au moins une amine tertiaire est nécessaire pour que la réaction ait lieu.

Parmi les amines tertiaires particulièrement utiles, on peut citer celles dont la ou les partie(s) aliphatique(s) ont chacune de 1 à 8 atomes de carbone et/ou la ou les partie(s) cycloaliphatique(s) ont chacune de 6 à 8 atomes de carbone, ces parties pouvant être substituées par des radicaux aromatiques, telles que la triéthylamine, la tributylamine, la diisopropyl éthylamine, la tricyclohexylamine, la N,N-di(phényléthyl)-méthylamine, les amines tertiaires dont un radical est constitué par un système cyclique aromatique telles que la N,N-diméthyl- ou N,N-diéthylaniline, la 4,4'-bis(diméthylamino)benzophénone appelée aussi cétone de Mischler, la N,N-diméthylaminopyridine, celles dont l'atome d'azote fait partie d'un hétérocycle, telles que la N-méthylmorpholine, le N-méthylimidazole.

On choisit généralement des amines tertiaires peu chères. La diisopropyléthylamine, la triéthylamine et la N-méthylmorpholine conviennent bien.

L'anhydride et l'acide de Meldrum sont généralement utilisés en quantité stoechiométrique et l'amine tertiaire en quantité supérieure à 2 équivalents par rapport à l'anhydride, de préférence en quantité voisine de 3 équivalents.

La réaction est effectuée dans un milieu solvant organique inerte. Comme solvants convenables, on peut citer les hydrocarbures aliphatiques ou aromatiques, chlorés ou non, tels que le dichlorométhane, le dichloro-1,2-éthane, le toluène, le chlorobenzène, les éthers cycliques ou non et les cétones. On emploie souvent le dichlorométhane ou le tétrahydrofurane.

De grandes quantités de solvants ne sont généralement pas nécessaires. On opère souvent dans un milieu réactionnel concentré.

Il est préférable que les constituants soient anhydres.

Contrairement à d'autres procédés, il n'est absolument pas nécessaire de refroidir le milieu réactionnel. La température ambiante, voisine de 20°C, convient bien pour réaliser le procédé.

Les dérivés dioxane-diones souhaités sont obtenus généralement en quelques minutes. Ils sont très facilement récupérés sous forme brute au moyen des traitements habituels tels que par lavages et évaporation des solvants. Ils peuvent ensuite être aisément transformés de façon connue en dérivés d'acides tétramiques. Si on le souhaite, on peut les purifier par des méthodes classiques.

Le procédé selon l'invention permet notamment de simplifier la synthèse des 4-amino-3-hydroxy-acides et de leurs dérivés par la voie des acides tétramiques, grâce à l'étape de préparation des dérivés dioxanes-diones qui s'effectue à partir de composés stables, qui ne nécessite pas l'emploi de réactifs chers ou difficiles à manipuler ni des conditions réactionnelles particulières et qui est rapide. Les acides tétramiques qui en dérivent sont obtenus avec de bons rendements et sont optiquement purs.

Les exemples qui suivent illustrent l'invention sans toutefois la limiter.

Dans tous les exemples, les dérivés de départ sont sous la forme (L). Il est bien évident que le procédé s'applique également à la préparation des composés avec la configuration opposée.

### Exemples 1 à 16

Les dérivés 5-(1-hydroxy-2-uréthaneéthylidène)-2,2-diméthyl-1,3-dioxane-4,6-diones (3) sont préparés selon le mode opératoire général suivant :

Un équivalent de UNCA (1) et un équivalent d'acide de Meldrum (2) sont mis à réagir dans du dichlorométhane (DCM) ou du tétrahydrofurane (THF) (2ml/mmol) à la température ambiante (voisine de 20° C) en présence de 3 équivalents de triéthylamine, de diisopropyléthylamine ou de N-méthylmorpholine. La réaction est suivie en chromatographie couche mince (C.C.M.) avec le système éluant suivant : acétate d'éthyle (AcOEt)/méthanol (MeOH)/acide acétique (Ac0H) : 95/3/2 (les rapports des éluants sont dans tout le texte exprimés en volume). Elle est généralement terminée au bout de quelques minutes. Le milieu réactionnel est repris par du DCM, lavé avec une solution aqueuse de KHSO₄ à 5% puis avec de l'eau. Il est ensuite séché sur sulfate de sodium et la phase organique est concentrée sous pression réduite pour obtenir le composé (3) désiré.

Afin de vérifier que les dérivés dioxane-diones obtenus conservent la configuration du produit de départ, on les transforme en dérivés d'acides tétramiques (4) puis ceux-ci en dérivés de 4-hydroxypyrrolidine-2-ones (5) de la façon suivante :

Le dérivé (3) brut précédemment obtenu est dissout dans du méthanol ou de l'acétate d'éthyle et la solution est placée à la température de reflux. La réaction est suivie par C.C.M. (AcOEt/Me0H/Ac0H : 95/3/2 ou AcOEt/hexane/Ac0H : 7/3/1). Elle est terminée en 1 à 2 heures. Après évaporation, le mélange réactionnel est repris par AcOEt et lavé par des solutions aqueuses de KHS0₄ à 5%, de bicarbonate de sodium saturée et de NaCl saturée. Après séchage et évaporation, on obtient le composé (4).

Pour le transformer en composé (5), il est repris dans un mélange DCM/Ac0H (10/1) et refroidi dans un bain de glace. On additionne ensuite petit à petit 2 équivalents molaires (8 équivalents hydrures) de NaBH4 en 1 heure environ dans le milieu réactionnel agité vigoureusement et on le maintient quelques heures à la même température. La réaction est suivie en C.C.M. (AcOEt/Me0H/Ac0H : 95/3/2 ou DCM/AcOEt : 7/3). Si nécessaire, on rajoute de l'hydrure. Le milieu réactionnel est ensuite hydrolysé par addition d'un mélange eau-glace. Après décantation, la phase organique est lavée à l'eau, séchée et évaporée. Le mélange résiduel est chromatographié sur silice avec le système solvant AcOEt/hexane : 5/5 ou DCM/AcOEt : 7/3 et on obtient le dérivé de 4-hydroxypyrrolidine-2-one (5) pur.

Les résultats obtenus à partir des N-carboxyanhydrides de différents amino-acides N-protégés sont rassemblés dans les tableaux I à III suivants. Les rendements en composés isolés sont calculés dans tous les exemples à partir des UNCA de départ, par rapport aux composés bruts obtenus dans les exemples 1 à 13 et par rapport aux composés purifiés obtenus dans les exemples 14 à 16.

Dans les exemples 5 à 13, les dérivés d'acides tétramiques (4) ont été préparés. Les pouvoirs rotatoires ont été mesurés sur les produits bruts. Les spectres de masse sont conformes aux structures attendues. Les spectres RMN ¹H (250MHz) confirment la structure des composés.

Dans les exemples 14 à 16, les dérivés de 4-hydroxypyrrolidine-2-ones (5) ont été préparés et purifiés. Les mesures effectuées montrent que la chiralité du composé de départ a été conservée.

Selon le même mode opératoire général, on a également préparé les dérivés de 4-hydroxypyrrolidine-2-ones (5) à partir des UNCA de Fmoc-L-Ala, Fmoc-L-Ile, Fmoc-L-Thr(OBut), Boc-L-Val, Boc-L-Ala, Z-L-Ala, Fmoc-L-Lys(Alloc) (Rendement 72 %), Fmoc-L-Met(Rendement : 80 %).

**TABLEAU I**

| Ex. | UNCA de l'amino-acide suivant : | Rendement en composé (3) % | Rf (éluant A) | Rf (éluant B) | Point de fusion ° C |
|---|---|---|---|---|---|
| 1 | Fmoc-L-Lys(Boc) | 82 | 0,6 | 0,5 | 121-123 |
| 2 | Fmoc-L-Lys(Alloc) | 86 | 0,6 | 0,6 | 93-95 |
| 3 | Fmoc-L-Thr(OBu) | 85 | 0,655 | 0,75 | 98-102 |
| 4 | Alloc-L-Val | 50 | 0,43 | 0,58 | 105-110 |
| Eluant A : CHCl₃/Me0H/Ac0H : 180/10/5 ; Eluant B : AcOEt/Me0H/Ac0H : 95/3/2. | | | | | |

**TABLEAU II**

| Ex. | UNCA de l'amino-acide suivant : | Rendement en composé (4) % | Rf (éluant A) | Rf (éluant B) | [α]²⁰_{D} (c 1,MeOH) | Spectre de masse, FAB+ |
|---|---|---|---|---|---|---|
| 5 | Fmoc-L-Phe | 84 | 0,45 | 0,57 | +104 | 412 |
| 6 | Boc-L-Phe | 80 | 0,37 | 0,49 | +205 | 290 |
| 7 | Z-L-Phe | 82 | 0,35 | 0,55 | +150 | 324 |
| 8 | Fmoc-L-Leu | 79 | 0,54 | | +66 | |
| 9 | Z-L-Leu | 76 | 0,51 | | +44 | |
| 10 | Fmoc-L-Val | 63 | 0,61 | 0,68 | +44 | 364 |
| 11 | Z-L-Val | 80 | 0,75 | 0,57 | +54 | 276 |
| 12 | Boc-L-Trp(For) | 60 | 0,37 | 0,35 | +113 | 357 |
| 13 | Fmoc-L-Lys(Boc) | 75 | 0,52 | 0,80 | +46 | 493 |
| Eluant A : CHCl₃/Me0H/Ac0H : 180/10/5 ; Eluant B : AcOEt/hexane/Ac0H : 7/3/1. For = formyl. | | | | | | |

**TABLEAU III**

| Ex. | UNCA de l'amino-acide suivant : | Rendement en composé (5) % | Rf (éluant A) | Rf (éluant B) | [α]²⁰_{D} (c 1,Me0H) | Spectre de masse, FAB+ |
|---|---|---|---|---|---|---|
| 14 | Boc-L-Leu | 65 | 0,50 | | +55 | 258 |
| 15 | Fmoc-L-Leu | 60 | | 0,65 | +42 | 379 |
| 16 | Z-L-Leu | 60 | | 0,46 | +58 | 292 |
| Eluant A : CHCl₃/Me0H/Ac0H : 180/10/5 ; Eluant B : DCM/AcOEt : 7/3. | | | | | | |

### Exemple 17 : Préparation de l'acide (3S,4S)-6-méthyl-4-t-butoxycarbonylamino-3-hydroxy-heptanoïque(Boc-statine).

Afin de s'assurer que le procédé de l'invention permet d'obtenir la statine et ses analogues avec la configuration souhaitée, on prépare la Boc-statine à partir de 2,49 g de l'UNCA de la Boc-L-Leucine, en utilisant le mode opératoire général décrit dans les exemples précédents. On obtient 1,62 g (Rendement 65%) de (4S,5S)-4-hydroxy-5-isobutyl-1-t-butoxycarbonylpyrrolidine-2-one dont les caractéristiques sont les suivantes : Point de fusion (F) = 92-93° C ; Rf = 0,58 (éluant : AcOEt/Hexane 75/25), (α)²⁰_{D} = +55(c1,Me0H) ; RMN ¹H(CDCl₃) δ ppm : 0,90(6H, dd, Me) 1,48(9H, s, Boc) 1,72(3H, m, CH et CH₂) 2,60(2H, qd, 3-H) 4,15(1H, m, 5-H) ; 4,50(1H, m, 4-H).

1 g de cette 4-hydroxypyrrolidine-2-one est dissout dans 5 ml d'acétone. On additionne goutte à goutte 2 ml de soude 1M. Après 2 heures, le mélange réactionnel est acidifié avec précaution avec de l'acide chlorhydrique dilué jusqu'au pH 3-4. La Boc-statine obtenue est précipitée par addition d'eau, essorée, lavée avec de l'eau, puis avec de l'hexane et séchée sous vide en présence de pastilles de potasse. On obtient ainsi 810 mg de cette statine, sous forme d'un solide blanc, ayant les caractéristiques suivantes : F = 120-120° C ; Rf = 0,52 (éluant CHCl₃/MeOH/AcOH : 180/10/5) ; (α)²⁰_{D} = 41,9(c1,MeOH) ; RMN ¹H(DMSO d6) δ ppm : 0,85(6H,dd,CH₃) ; 1,26(2H,m,CH₂) ; 1,37(9H,s,Boc) ; 1,55(1H,m,CH) ; 2,21(2H,m,2H) ; 3,51(1H,m,4-H) ; 3,80(1H,m,3-H) ; 6,31(1H,d,NH) ; SM-FAB+ : 276.

## Revendications

1. Procédé de préparation de dérivés de 5-(1-hydroxy-2-uréthane-éthylidène)-2,2-diméthyl-1,3-dioxane-4,6-diones au moyen de l'acide de Meldrum, caractérisé en ce qu'on fait réagir un N-carboxy-anhydride d'α-amino-acide N-uréthane protégé avec l'acide de Meldrum, dans un milieu solvant organique inerte, en présence d'une quantité supérieure à 2 équivalents par rapport au N-carboxy-anhydride d'au moins une amine tertiaire choisie parmi les amines tertiaires dont un radical au moins est aliphatique ou cycloaliphatique.

2. Procédé selon la revendication 1, caractérisé en ce que la ou les partie(s) aliphatique(s) de l'amine ont chacune de 1 à 8 atomes de carbone et la/ou les partie(s) cycloaliphatiques ont chacune de 6 à 8 atomes de carbone.

3. Procédé selon la revendication 1, caractérisé en ce que l'amine tertiaire est choisie parmi la diisopropyléthylamine, la triéthylamine et la N-méthylmorpholine.

4. Procédé selon l'une quelconque des revendications précédentes, caractérisé en ce que l'amine tertiaire est ajoutée en quantité voisine de 3 équivalents par rapport au N-carboxy-anhydride.

5. Procédé selon l'une quelconque des revendications précédentes, caractérisé en ce que l'anhydride et l'acide de Meldrum sont mis à réagir en quantité stoechiométrique.

6. Procédé selon l'une quelconque des revendications précédentes, caractérisé en ce que le groupe protecteur de la fonction amine de l'anhydride est choisi parmi les groupes (9-fluorénylméthyl)oxycarbonyle,tert-butyloxycarbonyle, benzyloxycarbonyle.

7. Procédé selon l'une quelconque des revendications 1 à 5, caractérisé en ce que le groupe protecteur de la fonction amine de l'anhydride est le groupe allyloxycarbonyle.

8. Procédé selon l'une quelconque des revendications précédentes, caractérisé en ce que les groupes fonctionnels portés par les chaînes latérales de l'α-amino-acide formant le N-carboxy-anhydride sont protégés.

9. Procédé selon l'une quelconque des revendications précédentes, caractérisé en ce que le milieu solvant est choisi parmi les hydrocarbures chlorés ou non, aliphatiques ou aromatiques, les éthers cycliques ou non et les cétones.

10. Procédé selon l'une quelconque des revendications précédentes, caractérisé en ce que les dérivés de 5-(1-hydroxy-2-uréthane-éthylidène)-2,2-diméthyl-1,3-dioxane-4,6-diones sont récupérés sous forme brute.

## Claims

1. Method for preparing derivatives of 5-(1-hydroxy-2-urethane-ethylidene)-2,2-dimethyl-1,3-dioxane-4,6-diones by means of Meldrum acid, characterized in that an N-carboxy anhydride of an N-urethane-protected α-amino acid is reacted with Meldrum acid, in an inert organic solvent medium, in the presence of a quantity greater than 2 equivalents with respect to the N-carboxy-anhydride of at least one tertiary amine chosen from tertiary amines of which at least one radical is aliphatic or cycloaliphatic.

2. Method according to claim 1, characterized in that each of the aliphatic part(s) of the amine has 1 to 8 carbon atoms and in that each of the cycloaliphatic part(s) has 6 to 8 carbon atoms.

3. Method according to claim 1, characterized in that the tertiary amine is chosen from diisopropylethylamine, triethylamine and N-methylmorpholine.

4. Method according to any one of the preceding claims, characterized in that the tertiary amine is added in a quantity approaching 3 equivalents with respect to the N-carboxy-anhydride.

5. Method according to any one of the preceding claims, characterized in that the anhydride and the Meldrum acid are reacted in a stoichiometric quantity.

6. Method according to any one of the preceding claims, characterized in that the group protecting the amine functional group of the anhydride is chosen from the groups (9-fluorenylmethyl)oxycarbonyl, tert-butyloxycarbonyl and benzyloxycarbonyl.

7. Method according to any one of claims 1 to 5, characterized in that the group protecting the amine functional group of the anhydride is the allyloxycarbonyl group.

8. Method according to any one of the preceding claims, characterized in that the functional groups carried by the side chains of the α-amino acid forming the N-carboxy-anhydride are protected.

9. Method according to any one of the preceding claims, characterized in that the solvent medium is chosen from aliphatic or aromatic chlorinated or non-chlorinated hydrocarbons, cyclic or non-cyclic ethers and ketones.

10. Method according to any one of the preceding claims, characterized in that the derivatives of 5-(1-hydroxy-2-urethane-ethylidene)-2,2-dimethyl-1,3-dioxane-4,6-diones are recovered in the crude form.

## Patentansprüche

1. Verfahren zur Herstellung von 5-(1-Hydroxy-2-methanethyliden)-2,2-dimethyl-1,3-dioxan-4,6-dionen mit Hilfe von Meldrumsäure,
gekennzeichnet durch
Umsetzung eines N-Carboxyanhydrids einer N-Urethan-geschützten α-Aminosäure mit Meldrumsäure in einem inerten organischen Lösungsmittelmedium in Gegenwart mindestens eines unter den tertiären Aminen, die mindestens einen aliphatischen oder cycloaliphatischen Substituenten aufweisen, ausgewählten tertiären Amins in einer Menge von mehr als 2 Äquivalenten, bezogen auf das N-Carboxyanhydrid.

2. Verfahren nach Anspruch 1, dadurch gekennzeichnet, daß die aliphatische(n) Gruppe(n) des Amins jeweils 1 bis 8 Kohlenstoffatome aufweisen und die cycloaliphatische(n) Gruppe(n) jeweils 6 bis 8 Kohlenstoffatome besitzen.

3. Verfahren nach Anspruch 1, dadurch gekennzeichnet, daß das tertiäre Amin unter Diisopropylethylamin, Triethylamin und N-Methylmorpholin ausgewählt ist.

4. Verfahren nach einem der vorhergehenden Ansprüche, dadurch gekennzeichnet, daß das tertiäre Amin in einer Menge zugegeben wird, die etwa 3 Äquivalente, bezogen auf das N-Carboxyanhydrid, beträgt.

5. Verfahren nach einem der vorhergehenden Ansprüche, dadurch gekennzeichnet, daß das Anhydrid und die Meldrumsäure in stöchiometrischer Menge eingesetzt werden.

6. Verfahren nach einem der vorhergehenden Ansprüche, dadurch gekennzeichnet, daß die Schutzgruppe der Aminfunktion des Anhydrids unter 9-Fluorenylmethyloxycarbonyl, t-Butyloxycarbonyl und Benzyloxycarbonyl ausgewählt ist.

7. Verfahren nach einem der Ansprüche 1 bis 5, dadurch gekennzeichnet, daß die Schutzgruppe der Aminfunktion des Anhydrids Allyloxycarbonyl ist.

8. Verfahren nach einem der vorhergehenden Ansprüche, dadurch gekennzeichnet, daß die an den Seitenketten der α-Aminosäure, die als Carboxyanhydrid vorliegt, befindlichen funktionellen Gruppen geschützt sind.

9. Verfahren nach einem der vorhergehenden Ansprüche, dadurch gekennzeichnet, daß das Lösungsmittelmedium unter aliphatischen und aromatischen Kohlenwasserstoffen, die gegebenenfalls chloriert sind, cyclischen und nichtcyclischen Ethern und Ketonen ausgewählt ist.

10. Verfahren nach einem der vorhergehenden Ansprüche, dadurch gekennzeichnet, daß die Derivate der 5-(1-Hydroxy-2-methanethyliden)-2,2-dimethyl-1,3-dioxan-4,6-dione in roher Form gewonnen werden.
